(19) Europäisches Patentamt
European Patent Office
Office européen des brevets

(11) Publication number: **0 164 320**
**A1**

(12) # EUROPEAN PATENT APPLICATION

(21) Application number: **85850073.9**

(22) Date of filing: **06.03.85**

(51) Int. Cl.⁴: **A 61 K 31/165**, A 61 K 31/155, A 61 K 31/445, C 07 C 129/12

(30) Priority: **14.03.84 SE 8401409**

(43) Date of publication of application: **11.12.85**
**Bulletin 85/50**

(84) Designated Contracting States: **AT BE CH DE FR GB IT LI LU NL SE**

(71) Applicant: **Astra Läkemedel Aktiebolag,**
**Strängnäsvägen 44, S-151 85 Södertälje (SE)**

(72) Inventor: **Post, Claes, Fatbursvägen 12,**
**S-150 30 Mariefred (SE)**

(74) Representative: **Hjertman, Ivan T. et al, AB ASTRA**
**Patent and Trade Mark Department, S-151 85 Södertälje (SE)**

(54) **Pharmaceutical preparations for spinal analgesia containing guanfacine, and method for their preparation.**

(57) A pharmaceutical preparation for spinal analgesia, which alleviates postoperative or chronic pain containing guanfacine or a physiologically acceptable salt thereof alone or together with a local anaesthetic, such as bupivacaine or a physiologically acceptable salt thereof, a method for obtaining said preparation and a method of obtaining analgesia by administering said preparation to a patient in the need of analgesia.

EP 0 164 320 A1

# New pharmaceutical preparations and a new method of alleviation of pain

## Field of the Invention

The present invention is related to new pharmaceutical preparations, a method for their preparation and to a new method of alleviation of postoperative pain and pain related to pathophysiological changes in man, such as severe chronic pain, wherein the new pharmaceutical preparations are used.

## Background of the Invention

The suffering from pain after operations, and that related to diseases as cancer and neurological diseases, has been the subject to treatment with e.g. opiate analgesics. These have several severe draw-backs, as e.g. development of tolerance, addiction and sedation with risk for respiratory depression. There is thus a need for analgesic agents intended for pain relief post-operatively and for relief of severe pain of other origins, which do not have the mentioned draw-backs.

In recent years, several possible alternative mechanisms for treatment of pain have become evident. One of these is described by Reddy and Yaksh. (Reddy, S.V.R. and Yaksh, T.L. Brain Res. 189, 391, 1980.) According to this reference noradrenaline causes a dose-dependent antinociceptive effect in rats and monkey. As the animals in this testing situation show an impaired responsiveness to thermal pain of the tail in the tail-flick (d'Amour, F.E. and Smith, D.L. J. Pharmacol. Exp. Ther. 72, 74, 1941) or the feet in the hot plate test (Eddy, N.B. and Leimbach, D. J. Pharmacol. Exp. Ther. 107, 385,) it is conceivable that this is a strong experimental support for an analgesic effect of the administered drug.

## Outline of the Invention

According to the present invention it has been found that the known compound guanfacine (2,6-dichlorophenyl-acetyl-guanidine), with the formula

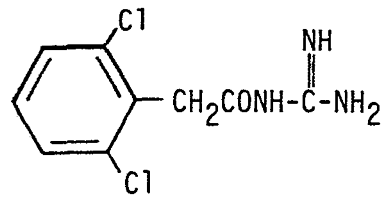

gives an antinociceptive effect when administered by intrathecal injection. Guanfacine thus can be used to alleviate pain when administered intrathecally or epidurally. Guanfacine is known as an antihypertensive agent and is described in the specification and claims of US-A-3 632 645, the contents of which are incorporated by reference herein.

Guanfacine or a physiologically acceptable salt thereof was administered alone or in combination with a local anaesthetic agent such as bupivacaine or a physiologically acceptable salt thereof.

Examples of physiologically acceptable salts, which are used according to this invention are hydrochloride, lactate, acetate or sulphamate.

Especially preferred physiologically acceptable salts are the hydrochlorides of guanfacine and bupivacaine.

The present invention thus provides new pharmaceutical preparations containing guanfacine and a local anaesthetic agent, methods for preparing such preparations and methods for alleviating pain sensation wherein said pharmaceutical preparation or a preparation containing guanfacine alone as active ingredient is intended to be used in the post-operative period or for the treatment of severe chronic pain. The pharmaceutical preparations are for spinal analgesia. They are preferably administered by intrathecal or epidural injection. In a preferred embodiment of this invention guanfacine is incorporated in a pharmaceutical preparation suitable for intrathecal injection.

In a further preferred embodiment of this invention guanfacine is incorporated in a pharmaceutical preparation suitable for intrathecal injection together with a local anaesthetic agent, such as bupivacaine, lidocaine or tetracaine, wherein bupivacaine is especially preferred.

A convenient route of administration of guanfacine is spinally i.e. intrathecally or epidurally, which will allow repeated administration over a long period of time. When administering the drug this way, a convenient way of restricting spread to segments more rostrally than intended, would be to make the solution hyperbaric by adding glucose to desired density or to add a vasoconstrictor such as adrenaline to the solution.

The dosage of administration of guanfacine depends i.a. on the route of administration and the pharmaceutical formulation. A suitable dosage for obtaining analgesia by intrathecal administration is between 0.001 and 0.5 mg/kg, and preferably higher when the preparation is administered epidurally.

Pharmaceutical preparations

In clinical practice guanfacine will normally be injected intrathecally or epidurally. The preparations used are solutions which contain between 0.001 and 1% by weight of the active compound guanfacine, and between 5-10% by weight of glucose in order to increase the baricity of the solution. It is also possible to add a small amount of the vasoconstrictor adrenaline to the solution in order to restrict spreading. When guanfacine is combined with bupivacaine, the bupivacaine concentration is between 0.25 and 0.75% by weight. The preparations are made by dissolving the active ingredient or ingredients in a physiologically acceptable solvent.

Example 1

| | |
|---|---|
| Guanfacine HCl | 5 mg/ml |
| Bupivacaine HCl | 5 mg/ml |
| Sodium chloride | 9 mg/ml |
| Distilled water | ad 100 ml |

Example 2

| | |
|---|---|
| Guanfacine HCl | 5 mg/ml |
| Bupivacaine HCl | 5 mg/ml |
| Glucose | 50 mg/ml |
| Sodium chloride | 9 mg/ml |
| Distilled water | ad 100 ml |

Example 3

| | |
|---|---|
| Guanfacine HCl | 5 mg/ml |
| Sodium chloride | 9 mg/ml |
| Distilled water | ad 100 ml |

Example 4

| | |
|---|---|
| Guanfacine HCl | 5 mg/ml |
| Glucose | 50 mg/ml |
| Sodium chloride | 9 mg/ml |
| Distilled water | ad 100 ml |

Example 5

| | |
|---|---|
| Guanfacine HCl | 5 mg/ml |
| Bupivacaine HCl | 5 mg/ml |
| Adrenaline | 0.005 mg/ml |
| Sodium chloride | 9 mg/ml |
| Distilled water | ad 100 ml |

Example 6

| | |
|---|---|
| Guanfacine HCl | 0.01 mg/ml |
| Bupivacaine HCl | 5 mg/ml |
| Sodium chloride | 9 mg/ml |
| Distilled water | ad 100 ml |

Example 7

| | |
|---|---|
| Guanfacine HCl | 0.06 mg/ml |
| Bupivacaine HCl | 5 mg/ml |
| Sodium chloride | 9 mg/ml |
| Distilled water | ad 100 ml |

Example 8

| | |
|---|---|
| Guanfacine HCl | 0.01 mg/ml |
| Sodium chloride | 9 mg/ml |
| Distilled water | ad 100 ml |

Example 9

| | |
|---|---|
| Guanfacine HCl | 0.06 mg/ml |
| Sodium chloride | 9 mg/ml |
| Distilled water | ad 100 ml |

Example 10

| | |
|---|---|
| Guanfacine HCl | 0.01 mg/ml |
| Glucose | 50 mg/ml |
| Sodium chloride | 9 mg/ml |
| Distilled water | ad 100 ml |

Example 11

| | |
|---|---|
| Guanfacine HCl | 0.06 mg/ml |
| Glucose | 50 mg/ml |
| Sodium chloride | 9 mg/ml |
| Distilled water | ad 100 ml |

Example 7 is considered to represent the best mode of carrying out the invention known at present.

Pharmacological tests

The antinociceptive effect of guanfacine may be established by the hot plate and tail-flick test.

Testing of tail-flick was performed using an IITC Inc. Mod. 33 Analgesiameter®, with the light focused on the tip of the tail (d'Amour, F.E. and Smith, D.L. J. Pharmacol. Exp. Ther. 72, 74, 1941). The intensity of the beam was adjusted to give a reaction time of 3-4 sec. before intrathecal injection of the drug solutions. For the hot plate testing, a modification of the Eddy and Leimbach method (Eddy, N.B. and Leimbach, D. J. Pharmacol. Exp. Ther. 107, 385, 1952) was used. An IITC Inc. Mod. 35-D Analgesiameter® was set at a temperature of 58±0.2°C. The criteria for a pain response was licking of either of the hindpaws.

We examined the pain responsiveness of male Sprague-Dawley rats in a weight range of 300-350 g. The animals had an indwelling catheter in the spinal subarachnoid space, which was inserted via the cisterna magna. The catheter was fixed at a surgical operation. The animals were kept in the animal colony in separate cages until testing took place 1 week after operation. The drug was administered in a volume of 15 µl, and testing of hot plate and tail-flick reaction latencies took place at the intervals given in the tables. Animals given the combination of guanfacine and bupivacaine were not tested until the local anaesthetic effect of bupivacaine had disappeared, which normally took between 10 and 15 minutes.

Guanfacine or a physiologically acceptable salt thereof was administered alone or in combination with the local anaesthetic agent bupivacaine or a physiologically acceptable salt thereof. When guanfacine was administered together with bupivacaine first an initial phase of regional anaesthesia restricted to the hind-legs and the lower dermatomes was obtained. When this effect had disappeared the animals were still non-responsive in the hot plate and tail-flick test for up to at least 7 hours after the injection, cf Table 2 below.

Table 1.

Antinociceptive effects after intrathecal injection of 75 μg guanfacine (15 μl 0.5 % guanfacine). Reaction latency was calculated according to the formula:

Antinociception=100x(test-latency - pre-drug latency)/pre-drug latency

The values are given as mean ± S.E.M. and the number of animals (Sprague-Dawley rats (Anticimex, Stockholm Sweden) in a weight range of 300-350 g) in each group was 4; n.d.=not determined.

| Time | tail-flick (cut-off=10 sec.) | hot plate (cut-off=30 sec.) |
|---|---|---|
| 1 min | 27.5±17 | -15±12 |
| 5 min | 92.75±26 | -15±6 |
| 10 min | 110±33 | 29±18 |
| 20 min | 147±13 | 62±29 |
| 30 min | 153±10 | 116±36 |
| 45 min | 156±8 | 239±23 |
| 8 hours | 138±20 | 124±56 |
| 22 hours | 62±8 | 41±12 |
| 27 hours | n.d. | 25±16 |

Table 2.

Antinociceptive effects after intrathecal injection of 75 μg guanfacine together with 75 μg bupivacaine (15 μl 0.5% guanfacine, 0.5% bupivacaine). Reaction latency was calculated according to the formula:

Antinociception=100x(test-latency - pre-drug latency)/pre-drug latency

The values are given as mean ± S.E.M. and the number of animals (Sprague-Dawley rats (Anticimex, Stockholm Sweden) in a weight range of 300-350 g) in each group was 4: m.b.=motor blockade due to bupivacaine.

| Time | tail-flick (cut-off=10 sec.) | hot plate (cut-off=30 sec.) |
|---|---|---|
| 1 min | m.b. | m.b. |
| 10 min | m.b. | m.b. |
| 20 min | 156±15 | 149±98 |
| 30 min | 148±12 | 174±88 |
| 45 min | 144±12 | 160±92 |
| 60 min | 156±15 | 226±95 |
| 7 hours | 132±17 | 184±86 |
| 22 hours | 35±24 | -20±39 |

Table 3.

Antinociceptive effects after intrathecal injection of vehicle. Reaction latency was calculated according to the formula:

Antinociception=100x(test-latency - pre-drug latency)/pre-drug latency

The values are given as mean ± S.E.M. and the number of animals (Sprague-Dawley rats (Anticimex, Stockholm Sweden) in a weight range of 300-350 g) in each group was 4.

| Time | tail-flick (cut-off=10 sec.) | hot plate (cut-off=30 sec.) |
|---|---|---|
| 1 min | 7±4 | 7±3 |
| 5 min | 16±3 | 13±7 |
| 15 min | 8±5 | -4±6 |
| 30 min | 10±7 | -3±5 |
| 45 min | 6±2 | -3±10 |
| 60 min | 19±8 | 0±9 |

As can be seen from Table 1, the reaction latency was prolonged both in the hot plate and tail-flick tests after intrathecally administered guanfacine and thus guanfacine is of great value to alleviate pain. After injection of the combination of bupivacaine and guanfacine, cf

Table 2, the animals were paralyzed in the hindlegs for up to between 10 and 15 min., wherefore the reaction latencies not were determined during this time. Thereafter, the same results were obtained as after guanfacine alone, i.e. the two agents appear to be additive in effects. As a comparison, the same volume of vehicle was injected, cf Table 3, and this was found to cause only very small and not significant changes in the reaction latencies. The vehicle used was a physiological solution of sodium chloride.

Tests on mice

Antinociceptive effects in mice were established in the following test. The effect 15 minutes after intrathecal injection of a volume of 5 µl containing different doses of guanfacine was established. The vehicle used was a physiological solution of sodium chloride.

Table 4.

Reaction latency was calculated according to the formula:

Antinociception=100x(test-latency - pre-drug latency)/pre-drug latency

The values are given as mean ± S.E.M.

| % Conc. of guanfacine | dose of guanfacine µg | tail-flick (cut-off=10 sec) | hot-plate (cut-off=30 sec) |
|---|---|---|---|
| 0.001 | 0.05 | 29±23 | 128±29 |
| 0.002 | 0.1 | 26±13 | 152±33 |
| 0.004 | 0.2 | 59±17 | 152±47 |
| 0.006 | 0.3 | 86±18 | 168±40 |

As can be seen from Table 4 the reaction latency was prolonged both in the hot-plate and the tail-flick test in mice at as low doses as 0.05 µg guanfacine.

Behavioural response in mice after subcutaneous injection of 20 µl of a physiological solution of sodium chloride or a 1 % solution of formaline on the dorsum of either hind paw.

5 µl, 0.01% (0.5 µg) of guanfacine or the same volume of a physiological solution of sodium chloride were injected intrathetecally 15 minutes before the subcutaneous injection of formaline. The accumulated reaction, i.e. the total time of licking on the injected paw, was measured during the 5 minutes after the injection of formaline.

The results are expressed as mean accumulated response during 5 minutes in seconds ± S.E.M. Each group consisted of 10 animals.

Table 5.

| | sec. ± S.E.M. |
|---|---|
| physiological solution of sodium chloride s.c. | 29±13 |
| 1% formaline s.c. + physiological solution of sodium chloride i.th. | 115±9 |
| 1% formaline s.c. + 5 µl of guanfacine i.th. | 9±2 |

Thus even pain caused by formaline, i.e. longlasting pain, is effectively blocked by guanfacine.

CLAIMS

1. A pharmaceutical preparation for spinal analgesia, characterized in that it contains at least one active ingredient guanfacine or a physiologically acceptable salt thereof as such or together with a local anaesthetic agent such as bupivacaine or tetracaine or a physiologically acceptable salt thereof.

2. A pharmaceutical preparation according to claim 1 characterized in that it also contains glucose.

3. A pharmaceutical preparation according to claim 1 characterized in that it also contains adrenaline.

4. A pharmaceutical preparation according to claims 1-3 characterized in that the only active ingredient is guanfacine or a physiologically acceptable salt thereof.

5. A pharmaceutical preparation according to claims 1-3 characterized in that the local anaesthetic agent is bupivacaine or a physiologically acceptable salt thereof.

6. A pharmaceutical preparation according to claims 2 and 5 characterized in that it contains 0.001-1% by weight of guanfacine, 0.25-0.75% by weight of bupivacaine and 5-10% by weight of glucose.

7. A method for the preparation of a pharmaceutical preparation according to claim 1 characterized in dissolving guanfacine or a physiologically acceptable salt thereof alone or together with a local anaesthetic agent or a physiologically acceptable salt thereof in a pharmaceutically acceptable solvent.

8. Use of guanfacine or a pharmaceutically acceptable salt thereof for the manufacture of a pharmaceutical preparation for intrathecal or epidural injection for obtaining analgesia.

9. Use of guanfacine or a physiologically acceptable salt thereof together with an amount of bupivacaine or a physiologically acceptable salt thereof effective to obtain local anaesthesia for the manufacture of a pharmaceutical preparation for intrathecal or epidural injection for obtaining analgesia.

10. Guanfacine or a physiologically acceptable salt thereof for use in alleviation of pain.

CLAIMS FOR AUSTRIA

1. A process for the preparation of a pharmaceutical preparation for spinal analgesia, characterized in dissolving at least one active ingredient guanfacine or a physiologically acceptable salt thereof as such or together with a local anaesthetic agent such as bupivacaine or tetracaine or a physiologically acceptable salt thereof in a pharmaceutically acceptable solvent.

2. A process according to claim 1 characterized in that also glucose is dissolved.

3. A process according to claim 1 characterized in that also adrenaline is dissolved.

4. A process according to claims 1-3 characterized in that the only active ingredient is guanfacine or a physiologically acceptable salt thereof.

5. A process according to claims 1-3 characterized in that the local anaesthetic agent is bupivacaine or a physiologically acceptable salt thereof.

6. A process according to claims 2 and 5 characterized in that the preparation obtained contains 0.001-1% by weight of guanfacine, 0.25-0.75% by weight of bupivacaine and 5-10% by weight of glucose.

7. Use of guanfacine or a pharmaceutically acceptable salt thereof for the manufacture of a pharmaceutical preparation for intrathecal or epidural injection for obtaining analgesia.

8. Use of guanfacine or a physiologically acceptable salt thereof together with an amount of bupivacaine or a physiologically acceptable salt thereof effective to obtain local anaesthesia for the manufacture of a pharmaceutical preparation for intrathecal or epidural injection for obtaining analgesia.

European Patent Office

# EUROPEAN SEARCH REPORT

0164320

Application number

EP 85 85 0073

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BE-A-893 276 (SANDOZ S.A.) *Page 3, lines 4-22; page 5, lines 21-24; example 2* --- | 1,7,8, 10 | A 61 K 31/165<br>A 61 K 31/155<br>A 61 K 31/445<br>C 07 C 129/12 |
| A | DE-A-1 806 400 (ESPE FABRIK PHARMAZEUTISCHER PRÄPARATE GMBH) *Claims* --- | 3 | |
| A | FR-M-3185 (SOCIÉTÉ DES USINES CHIMIQUES RHÔNE-POULENC) --- | 1 | |
| D,X | US-A-3 632 645 (DR. A. Wander S.A.) *Column 3, lines 52-75* --- | 1,7,10 | |
| X | US-A-4 046 917 (SANDOZ LTD) --- | 1,7,8, 10 | **TECHNICAL FIELDS SEARCHED (Int. Cl.4)** |
| X | US-A-4 404 226 (SANDOZ LTD) *Column 2, line 51-column 3, line 2; example 2* --- | 1,7, 10 | A 61 K<br>C 07 C |
| X | BRAIN RESEARCH, vol. 241, no. 2, June 1982, pages 393-397, Amsterdam (NL); J PAPANICOLAOU et al: "The relationship between $\alpha_2$ -adrenoceptor selectivity and anti-convulsant effect in a series of clonidine - like drugs". *Page 394, column 1* --- | 1,7, 10 | |

The present search report has been drawn up for all claims

| Place of search | Date of completion of the search | Examiner |
|---|---|---|
| STOCKHOLM | 11-06-1985 | HJÄLMDAHL M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0164320

Application number

European Patent Office

# EUROPEAN SEARCH REPORT

EP 85 85 0073

Page 2

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| P,X | BRAIN RESEARCH, vol. 309, no. 1, August 1984, pages 135-142, Amsterdam (NL);M.H.OSSIPOV et al: "Effect of $\alpha_2$ adrenergic agents upon central etorphine antinociception in the cat". *Page 135, first paragraph; page 136, column 1, lines 5-9* --- | 1,7,8, 10 | |
| X | CHEMICAL ABSTRACTS, vol. 96, No. 5, February 1,1982, page 52, column 2, abstract no. 28 504y, Columbus, Ohio (US); H. DEPOORTERE: "Sleep induction and central effects of some $\alpha$-adrenoceptor agonists"; & Sleep (Basel) 1980, 5 th, 283-6 *Abstract* --- | 1,7,10 | TECHNICAL FIELDS SEARCHED (Int. Cl.4) |
| A | ROTE LISTE 1977/78, BUNDESVERBAND DER PHARMAZEUTISCHEN INDUSTRIE, 1978, Editio Cantor, Aulendorf (DE). *No. 58 023B "Carbostesin® Inj.--Lsg. 0,25% / 0,5% mit Adrenalin 1:200 000"; No. 58 040B "Scandicain® 4% hyperbar" * ---- | 2,3 | |